# EUROPÄISCHE PATENTANMELDUNG

(11) **EP 4 190 312 A1**
(43) Veröffentlichungstag der Anmeldung: **07.06.2023**
(21) Anmeldenummer: 21212525.6
(22) Anmeldetag: 06.12.2021
(51) Int. Cl.: A61K 9/16, A61K 9/50, A61K 9/51, A61K 47/52, A61K 31/00, A61K 45/06

(54) **SYNERGISTISCHER TRANSPORT LIPOPHILER UND HYDROPHILER WIRKSTOFFE IN NANOPARTIKELN**

(71) Anmelder: Karlsruher Institut für Technologie, 76131 Karlsruhe (DE); Max-Planck-Gesellschaft zur Förderung der Wissenschaften e.V., 80539 München (DE); Georg-August-Universität Göttingen Stiftung Öffentlichen Rechts, Universitätsmedizin, 37075 Göttingen (DE)
(72) Erfinder: RUDOLPH, David, 75210 Keltern/Niebelsbach (DE); FELDMANN, Claus, 76275 Ettlingen (DE); NAPP, Joanna, 37124 Rosdorf (DE); ALVES, Frauke, 37075 Göttingen (DE)
(74) Vertreter: Müller-Boré & Partner Patentanwälte PartG mbB

(57) **Zusammenfassung**

Die vorliegende Erfindung betrifft Nanocontainer für den synergistischen Transport von lipophilen und hydrophilen Wirkstoffen bzw. Nachweisreagenzien. Insbesondere bieten die erfindungsgemäßen Nanocontainer eine Möglichkeit zur Diagnostik und/oder Behandlung von Krankheiten mit Kombinationen von Wirkstoffen (Therapie) und Nachweisreagenzien (Diagnostik), welche verschiedene Löslichkeitseigenschaften aufweisen können. Des Weiteren betrifft die vorliegende Erfindung ein Verfahren zur Herstellung der erfindungsgemäßen Nanocontainer.

## Beschreibung

Die vorliegende Erfindung betrifft Nanocontainer für den synergistischen Transport von lipophilen und hydrophilen Wirkstoffen bzw. Nachweisreagenzien. Insbesondere bieten die erfindungsgemäßen Nanocontainer eine Möglichkeit zur Diagnostik und/oder Behandlung von Krankheiten mit Kombinationen von Wirkstoffen (Therapie) und Nachweisreagenzien (Diagnostik), welche verschiedene Löslichkeitseigenschaften aufweisen können. Des Weiteren betrifft die vorliegende Erfindung ein Verfahren zur Herstellung der erfindungsgemäßen Nanocontainer.

Lipophile Verbindungen, insbesondere pharmazeutisch aktive Wirkstoffe, sind häufig von einer effektiven klinischen Anwendung ausgeschlossen, da diese nicht oder nur sehr schwer verabreicht werden können und/oder den Wirkort nur in unzureichender Konzentration erreichen. Dies ist insbesondere der Fall, wenn lipophile Verbindungen intravenös über die Blutbahn verabreicht werden oder wenn lipophile Verbindungen in wässriges Milieu eingebracht werden sollen (z.B. intravenöse oder intraperitoneale Verabreichung). Darüber hinaus ist die Zellaufnahme bzw. der Transport durch Membranen für lipophile Verbindungen häufig gegenüber hydrophilen Verbindungen stark herabgesetzt.

Nanocontainer bzw. Nanopartikel bieten hierbei eine bekannte Plattform für den Transport von pharmazeutisch aktiven Wirkstoffen, beispielsweise Chemotherapeutika für die Behandlung von Tumorerkrankungen. Die Nanocontainer unterstützen dabei die direkte und geschützte Medikamentenabgabe in den Tumor und können damit die Wirksamkeit des Chemotherapeutikums verbessern und/oder mögliche Nebenwirkungen vermeiden. In der Onkologie zählen hierzu nicht-PEGyliertes liposomales Doxorubicin (Myocet^{®}) oder PEGyliertes liposomales Doxorubicin (Caelyx^{®}), welche eine verbesserte Kardiotoxizität, Neutropenie und/oder Alopezie im Vergleich zum freien Wirkstoff zeigen. Des Weiteren ermöglichen PEGyliertes liposomales Irinotecan (Onivyde^{®}) oder nanopartikuläres Albumin-gebundenes Paclitaxel (Abraxane^{®}) den Einsatz von hochhydrophoben und hochpotenten Taxanen, die so in höherer Dosis, in kürzerer Zeit und ohne Co-Medikation verabreicht werden können.

Nachteile von liposomalen Nanocontainern bzw. Nanopartikeln stellen unter anderem die kurze Halbwertszeit und Stabilität in Suspension dar. Des Weiteren sind diese kostspielig in der Produktion und die Sterilisation ist wegen der Empfindlichkeit bei hohen Temperaturen und Strahlungsarten nur bedingt möglich.

Deshalb wurden weitere Nanopartikel-basierte Konzepte für den Transport von Chemotherapeutika durch die Materialwissenschaften vorgeschlagen. Diese Konzepte basieren auf organischen Matrices, beispielsweise Polymere oder Biopolymere, oder anorganischen Matrices, beispielsweise Siliziumdioxid, Eisenoxid oder Metallphosphate, in welche der pharmazeutische Wirkstoff eingebettet wird.

Ein Nachteil hierbei sind toxische und unter physiologischen Bedingungen nur schwer abbaubare Bestandteile, wodurch es zum Auftreten von erheblichen Nebenwirkungen kommen kann. Insbesondere für Siliziumdioxid wurde inzwischen eine karzinogene Langzeitwirkung gezeigt.

Des Weiteren wird der Wirkstoff nur oberflächlich an oder in die organische oder anorganische Matrix gebunden. Zudem ist die Menge an Wirkstoff bezogen auf die Gesamtmasse der Nanopartikel mit der Matrix als Mehrheitskomponente meist gering (< 20%). Lipophile Wirkstoffe zeigen zudem eine schlechte Stabilität in Suspension, was eine zu schnelle oder zu langsame Freisetzung des Wirkstoffes zur Folge hat. Weiter bieten solche Matrices nur eingeschränkt die Möglichkeit zum Transport von lipophilen, pharmazeutischen aktiven Wirkstoffen sowie zur Kombination von verschiedenen Wirkstoffen. Deshalb werden derartige Systeme trotz eines komplexen Materialsystems meist auch nur gegen ein Krankheitsbild verwendet. Aufgrund dessen beschränkten sich die Studien mit organischen und anorganischen Matrices für den Transport von pharmazeutisch aktiven Wirkstoffen bislang in den meisten Fällen lediglich auf *in vitro* Experimente.

Es besteht somit ein Bedarf an neuen Konzepten, die eine kostengünstige und effiziente Möglichkeit bieten, lipophile Verbindungen, beispielsweise pharmazeutisch aktive Wirkstoffe oder Nachweisreagenzien, als Kombinationspräparat mit hydrophilen Verbindungen, beispielsweise pharmazeutisch aktiven Wirkstoffen oder Nachweisreagenzien, bereitzustellen, um geringere Dosierungen und kürzere Behandlungsdauern zu erreichen und Nebenwirkungen zu vermeiden.

Die vorstehend beschriebene Aufgabe wird durch die in den Ansprüchen gekennzeichneten Ausführungsformen der vorliegenden Erfindung gelöst.

Insbesondere wird erfindungsgemäß ein Nanocontainer bereitgestellt, umfassend einen lipophilen Kern,
wobei der lipophile Kern mindestens eine lipophile Verbindung umfasst, ausgewählt aus einem lipophilen, pharmazeutisch aktiven Wirkstoff oder einem lipophilen Nachweisreagenz; und
eine hydrophile Hülle bzw. Schale, welche den lipophilen Kern umschließt,
wobei die hydrophile Schale aus einer anorganisch-organischen Hybridverbindung als lonenverbindung aufgebaut ist, wobei die anorganisch-organische Hybridverbindung aus einem anorganischen Metallkation, ausgewählt aus Mn²⁺, Sc³⁺, Y³⁺, La³⁺, Fe²⁺, Fe³⁺, [ZrO]²⁺, [HfO]²⁺, Bi³⁺, Gd³⁺ oder einem Lanthanoid Ln²⁺ oder Ln³⁺ oder einer hydratisierten Form dieser Kationen (z.B. [Gd(H₂O)ₙ]³⁺ mit n = 2-8, [Gd(OH)]²⁺, [GdO]⁺), und einem wasserlöslichen organischen Wirkstoffanion oder einem wasserlöslichen Nachweisreagenzanion, das jeweils mindestens eine Phosphat-, Phosphonat-, Sulfat-, Sulfonat-, Carbonat- oder Carboxylatgruppe als funktionelle Gruppe enthält, aufgebaut ist.

Wie vorstehend beschrieben, ist der lipophile Kern des erfindungsgemäßen Nanocontainers aus mindestens einer lipophilen Verbindung gebildet bzw. aufgebaut, wobei die lipophile Verbindung keiner besonderen Beschränkung unterliegt. Erfindungsgemäß werden unter dem Begriff "lipophile Verbindung" Verbindungen verstanden, die im Wesentlichen in Wasser schwer löslich (< 0,1 mol L⁻¹) und gut löslich (> 1,0 mol L⁻¹) in Alkanen, beispielsweise Hexan oder Dodecan und/oder aromatischen Kohlenwasserstoffen, beispielsweise Toluol, sind. Der lipophile Kern kann entweder eine oder mehrere lipophile Verbindungen umfassen.

Gemäß der vorliegenden Erfindung ist die mindestens eine lipophile Verbindung aus einem lipophilen, pharmazeutisch aktiven Wirkstoff oder einem lipophilen Nachweisreagenz ausgewählt. Unter dem Begriff "pharmazeutisch aktiver Wirkstoff" wird erfindungsgemäß ein Stoff verstanden, der als Mittel zur Heilung oder zur Verhütung menschlicher oder tierischer Krankheiten verwendet wird, sowie ein Stoff, der dazu bestimmt ist, im oder am menschlichen oder tierischen Körper zur Wiederherstellung, Besserung oder Beeinflussung der menschlichen oder tierischen Körperfunktionen angewendet zu werden. Unter dem Begriff "Nachweisreagenz" wird erfindungsgemäß ein Stoff bzw. eine Verbindung verstanden, der bzw. die nach Verabreichung im Körper detektiert/lokalisiert werden kann, beispielsweise optisch über Fluoreszenz im Falle eines Fluoreszenzfarbstoffs oder aber auch durch Röntgenabsorption, magnetische Messungen oder anhand deren radioaktiver Strahlung. Unter Umständen kann eine Verbindung gleichzeitig Wirkstoff und Nachweisreagenz sein. Beispielsweise zeigt das Chemotherapeutikum Irinotecan selbst blaue Fluoreszenz, die Zytostatika der Anthracyclin-Gruppe zeigen in der Regel Fluoreszenz, so fluoresziert zum Beispiel Doxorubicin rot, und das Virostatikum Dolutegravir zeigt rote Fluoreszenz.

Gemäß der vorliegenden Erfindung wird der lipophile Kern von einer Schale auf Basis einer anorganisch-organischen Hybridverbindung umschlossen. Durch diese Struktur wird der lipophile Kern stabilisiert und als Transport- und Lagerform verfügbar gemacht.

Die Schale des erfindungsgemäßen Nanocontainers umfasst bzw. besteht bzw. ist aufgebaut aus mindestens einer anorganisch-organischen Hybridverbindung, die als lonenverbindung wiederum aus einem anorganischen Metallkation und einem wasserlöslichen, organischen Anion, das ein organisches Wirkstoffanion und/oder ein hydrophiles Nachweisreagenzanion ist, aufgebaut ist.

Gemäß der vorliegenden Erfindung ist das anorganische Metallkation der hydrophilen Schale ausgewählt aus der Gruppe, bestehend aus Mn²⁺, Sc³⁺, Y³⁺,La³⁺, Fe²⁺, Fe³⁺, [ZrO]²⁺, [HfO]²⁺, Bi³⁺, Gd³⁺ oder einem Lanthanoid Ln²⁺ oder Ln³⁺ oder einer hydratisierten Form dieser Kationen (z.B. [Gd(H₂O)ₙ]³⁺ mit n = 2-8, [Gd(OH)]²⁺, [GdO]⁺), oder Gemischen davon. Besonders bevorzugt ist das anorganische Kation ausgewählt aus der Gruppe, bestehend aus Gd³⁺, [Gd(OH)]²⁺, [GdO]⁺ und [ZrO]²⁺

Durch die Auswahl bestimmter Metallkationen, ausgewählt aus der vorstehend definierten Liste, kann der Nanocontainer mit zusätzlichen Eigenschaften ausgestattet werden. Insbesondere durch die Auswahl schwerer, magnetischer und/oder radioaktiver anorganischer Metallkationen, beispielsweise [ZrO]²⁺, [⁸⁹ZrO]²⁺, ²²⁵Ac³⁺, [HfO]²⁺, Bi³⁺, Gd³⁺ oder Mn²⁺, kann eine Detektion der Nanocontainer durch Röntgenabsorption, magnetische Messungen und/oder durch radioaktiven Zerfall erfolgen.

Gemäß der vorliegenden Erfindung ist die anorganisch-organische Hybridverbindung eine lonenverbindung, die ein hydrophiles organisches Wirkstoffanion oder ein hydrophiles Nachweisreagenzanion umfasst. Dabei entsprechen die Definitionen des "Wirkstoffanions" und des "Nachweisreagenzanions" den vorstehend genannten Definitionen bezüglich des "pharmazeutisch aktiven Wirkstoffes" und des "Nachweisreagenz".

Des Weiteren enthält das wasserlösliche organische Wirkstoffanion oder das wasserlösliche Nachweisreagenzanion jeweils mindestens eine Phosphat-, Phosphonat-, Sulfat-, Sulfonat-, Carbonat- oder Carboxylatgruppe als funktionelle Gruppe, um derart zusammen mit dem anorganischen Metallkation die anorganisch-organische Hybridverbindung als lonenverbindung zu bilden, welche die den lipophilen Kern umschließende Schale der erfindungsgemäßen Nanocontainer bildet. Die anorganisch-organische Hybridverbindung an sich ist in Wasser schwer löslich.

Gemäß einer bevorzugten Ausführungsform der vorliegenden Erfindung ist der mindestens eine lipophile, pharmazeutisch aktive Wirkstoff ausgewählt aus
der Gruppe der Antibiotika, bestehend aus Delamanid, Bedaquilin, Benzothiazinonen wie Benzothiazinon 043, Clofazimin, Rifampicin, Levofloxacin, Cefaclor, Cefpodoxim, Imipenem, Meropenem, Ciprofloxacin, Levofloxacin, Norfloxacin, Chloramphenicol, Trimethoprim, Azithromycin, Metronidazol, Linezolid, Tyrothricin, Rifabutin, Rifaximin, Fusidinsäure, Doxycyclin, Hydroxytamoxifen und Pantoprazol; oder
der Gruppe der Virostatika, bestehend aus Amantadin, Rimantadin, Penciclovir, Emivirin, FGI-106, Maraviroc, Sofosbuvir, Baloxavirmarboxil, Etravirin, Nevirapin, Atazanavir, Indinavir, Lopinavir, Nelfinavir, Tipranavir, Boceprevir, Telaprevir, Dolutegravir, Raltegravir und Tecovirimat; oder
der Gruppe der Chemotherapeutika, bestehend aus Ifosfamid, Paclitaxel, Docetaxel, Abraxan, Taxoter, Mechlorethamin, Erlotinib, Gefitinib, Imatinib, Vemurafenib, Cisplatin, Daunorubicin, Epirubicin, Lomustin, Vismodegib, Actinomycin D, Vinorelbin, Camptothecin, Topotecan, Irinotecan, Etoposid, Teniposid und Mercaptopurin; oder der Gruppe der Entzündungshemmer, bestehend aus Cannabidiol, Triamcinolon, Budesonid, Diclofenac, Cortisol, Calcitriol, Leflunomid und nichtsteroidalen Antiphlogistika.

Des Weiteren ist gemäß einer weiteren bevorzugten Ausführungsform der vorliegenden Erfindung das Nachweisreagenz aus der Gruppe der Fluoreszenzfarbstoffe, bestehend aus Lumogen Rot, Lumogen Orange, Lumogen Gelb oder Lumogen Grün, Magnesiumphthalocyanin, Zinkphthalocyanin, 1,1'-Diethyl-4,4'-carbocyaniniodid, 3,3'-Diethylthiadicarbocyaniniodid, Magnesiumtetraphenylporphyrin und Phthalocyanin, ausgewählt.

Gemäß einer bevorzugten Ausführungsform der vorliegenden Erfindung beträgt die Masse des lipophilen, pharmazeutisch aktiven Wirkstoffes und/oder des lipophilen Nachweisreagenz 50 bis 100 Gew.-%, bezogen auf die Gesamtmasse des lipophilen Kerns, bevorzugt mindestens 60 Gew-%, besonders bevorzugt mindestens 70 Gew.-% und am meisten bevorzugt mindestens 75 Gew.-%. Da vom Trägersystem des Nanocontainers, d.h. von den weiteren Bestandteilen außer dem Wirkstoff, üblicherweise keine pharmazeutische Wirkung ausgeht, kann mit dem erfindungsgemäßen Nanocontainer eine große Beladungsmenge an Wirkstoff realisiert werden, so dass eine sehr hohe pharmazeutische Wirksamkeit pro verabreichter Nanocontainermenge erzielt werden kann.

Gemäß einer Ausführungsform der vorliegenden Erfindung beträgt die Masse des lipophilen, pharmazeutisch aktiven Wirkstoffes weniger als 100 Gew.-%, wenn der lipophile Kern einen oder mehrere lipophile Hilfsstoffe umfasst.

Unter "lipophilen Hilfsstoffen" werden Verbindungen verstanden, welche vorteilhaft auf die Eigenschaften des lipophilen Kerns wirken. Beispielhaft kann hier α-Tocopherol genannt werden, welches eine antioxidative Eigenschaft aufweist und damit zu einer verbesserten Stabilität führen kann. Weitere lipophile Hilfsstoffe können zum Beispiel Toluol, Phellandren oder natürliche Öle wie Ölsäure oder Linolensäure sein.

Gemäß einer Ausführungsform der vorliegenden Erfindung weist der lipophile Kern einen Durchmesser von 10 bis 150 nm, gemessen mittels Elektronenmikroskopie, auf. Bevorzugt weist der lipophile Kern einen Durchmesser von mindestens 10 nm, weiter bevorzugt von mindestens 20 nm und am meisten bevorzugt von mindestens 30 nm auf. Ein Durchmesser, der unter dem vorstehend genannten Minimum liegt, ist technisch schwer realisierbar.

Weiter bevorzugt weist der lipophile Kern einen Durchmesser bevorzugt von höchstens 120 nm, weiter bevorzugt von höchsten 80 nm und am meisten bevorzugt von höchstens 50 nm auf.

Wie vorstehend ausgeführt, wird die Schale des Nanocontainers aus einer anorganisch-organischen Hybridverbindung gebildet. Diese anorganisch-organische Hybridverbindung ist neben dem Metallkation aus einem hydrophilen organischen Wirkstoffanion oder einem hydrophilen Nachweisreagenzanion aufgebaut.

Gemäß einer bevorzugten Ausführungsform der vorliegenden Erfindung ist das hydrophile Wirkstoffanion bzw. das hydrophile Nachweisreagenzanion ausgewählt aus der Gruppe der Antibiotika, bestehend aus Clindamycinphosphat, Erythromycinphosphat, Tedizolidphosphat, CpG-Oligodesoxynukleotide, Fosfomycin, Moxalactam, Ceftriaxon, Amoxicillin, Phenoxymethylpenicillin, Aztreonam, Moxifloxacin und Bacitracin; oder
der Gruppe der Virostatika, bestehend aus Idoxuridinphosphat, Aciclovirphosphat, Penciclovirphosphat, Ganciclovirphosphat, Remdesivirphosphat, Galidesivirphosphat, Vidarabinphosphat, Ribavirinphosphat, Abacavirphosphat, Stavudinphosphat, Adefovir, Fosamprenavir, Fostemsavir, Tenofovir, Brincidofovir, Cidofovir, Foscarnet, Ivermectin, Bevirimat und Zanamivir, oder
der Gruppe der Chemotherapeutika, bestehend aus 5-Fluor-2'-desoxyuridin-5'-monophosphat, Gemcitabinmonophosphat, Gemcitabintriphosphat, Fludarabin, Pemetrexed, Methotrexat, Estramustinphosphat, Streptozotocinphosphat, Mitoxantronphosphat, Azacitidinphosphat, Cyclophosphamid Mustard, SN-38, Melphalan, Chlorambucil und Bendamustin; oder
der Gruppe der Entzündungshemmer, bestehend aus Betamethasonphosphat, Dexamethasonphosphat, Prednisolonphosphat, Sulfasalazin, Acetylsalicylat, Methotrexat, Ibuprofen, Naproxen und Ketoprofen; oder
der Gruppe der Fluoreszenzfarbstoffe, bestehend aus Phenylumbelliferonphosphat, Flavinmononukleotid, Methylfluorescinphosphat, Resorufinphosphat, Dynomics-546-uridintriphosphat, Dynomics-647-uridintriphosphat, Amaranth Rot, Chicago Sky Blue, Direktblau 71, Kongorot, Nuclear Fast Red, Acid Red 97 und Evens Blue.

Was die vorstehend aufgeführten Verbindungen betrifft, so liegen diese als Anion vor. Dabei werden die üblicherweise verwendeten Wirkstoffnamen verwendet, d.h. Clindamycinphosphat (obwohl die Ausgangsverbindung nicht das Anion, sondern die Säure oder das Natriumsalz ist), Ibuprofen (als Ausgangsverbindung korrekt; als Wirkstoff in den Nanopartikeln liegt aber das Anion vor), etc.. Einem Fachmann ist bekannt, dass das entsprechende Anion durch Lösen der Säure oder des Natriumsalzes in Wasser entsteht bzw. erzeugt werden kann.

Im Falle von SN-38, welches an sich keine der erfindungsgemäß definierten funktionalen Gruppen aufweist, muss die wässrige Lösung davon alkalisch eingestellt werden, so dass der innere cyclische Ester öffnet und eine freie Carboxyl-Funktion entsteht.

Gemäß einer Ausführungsform der vorliegenden Erfindung beträgt die Masse des hydrophilen, pharmazeutisch aktiven Wirkstoffes und/oder des Nachweisreagenz 50 bis 90 Gew.-%, bezogen auf die Gesamtmasse an organischen Anionen in der Schale, bevorzugt mindestens 60 Gew-%, besonders bevorzugt mindestens 70 Gew.-% und am meisten bevorzugt mindestens 75 Gew.-%. Da vom Trägersystem des Nanocontainers, d.h. von den weiteren Bestandteilen außer dem Wirkstoff, üblicherweise keine pharmazeutische Wirkung ausgeht, kann mit dem erfindungsgemäßen Nanocontainer eine große Beladungsmenge an Wirkstoff realisiert werden, so dass eine sehr hohe pharmazeutische Wirksamkeit pro verabreichter Nanocontainermenge erzielt werden kann.

Werden beispielsweise ein lipophiler, pharmazeutisch aktiver Wirkstoff im Kern und ein (hydrophiles) Nachweisreagenz in der Hülle bzw. Schale eingesetzt, kann der erfindungsgemäße Nanocontainer vorteilhafterweise den Wirkstoff nach der Darreichung freisetzen und durch das Nachweisreagenz in z.B. Zellen, Geweben und Organen lokalisiert werden.

Gemäß einer weiteren Ausführungsform der vorliegenden Erfindung weist der Nanocontainer einen Durchmesser von 20 bis 300 nm, gemessen mittels Elektronenmikroskopie, auf. Besonders bevorzugt weist der Nanocontainer einen Durchmesser von 30 nm oder mehr und am meisten bevorzugt von 50 nm oder mehr auf. Liegt der Durchmesser der Nanocontainer über der vorstehend genannten Untergrenze, weist der Nanocontainer eine vorteilhafte Stabilität auf und eine ausreichende Wirkstoffmenge pro Nanocontainer.

Weiter bevorzugt weist der Nanocontainer einen Durchmesser von 250 nm oder weniger, weiter bevorzugt von 150 nm oder weniger und am meisten bevorzugt von 100 nm oder weniger auf.

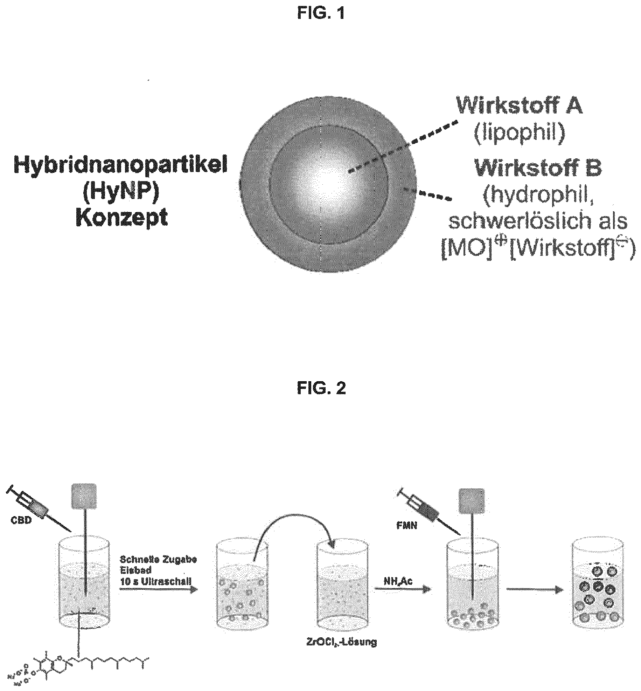

Ein weiterer Aspekt der vorliegenden Erfindung betrifft ein Verfahren zur Herstellung derartiger Nanocontainer. Die Herstellung der Kern-Schale-Partikel erfolgt dabei im Wesentlichen durch die Solvens-Antisolvens-Methode (siehe Figur 2). Hierbei wird zunächst eine hochkonzentrierte, möglichst gesättigte Lösung des lipophilen Wirkstoffes bzw. Nachweisreagenz in einem geeigneten Lösungsmittel hergestellt. Diese Solvens-Lösung wird unter starkem Rühren und/oder intensiver Ultraschalldurchmischung möglichst schnell in ein polares Antisolvens injiziert. Bevorzugt handelt es sich hierbei um Wasser bzw. um ein Gemisch von Wasser mit anderen, mit Wasser mischbaren Lösungsmitteln. Voraussetzung ist, dass sich der Wirkstoff im Solvens sehr gut, im Antisolvens dagegen sehr schlecht löst. Das Solvens muss weiterhin mit dem Antisolvens zumindest in einem gewissen Konzentrationsbereich mischbar sein. Die Injektion der Solvens-Lösung in das Antisolvens führt dann zum Ausfällen des lipophilen Wirkstoffes bzw. Nachweisreagenz unter Bildung von Nanopartikeln. Nachfolgend wird auf den Nanopartikeln, bestehend aus dem lipophilen Wirkstoff bzw. Nachweisreagenz, die Schale auf Basis der anorganisch-organischen Hybridverbindung abgeschieden (siehe Figur 2). Hierzu wird in der Regel das organische funktionelle Anion als erstes zugegeben. Nachfolgend wird die Lösung· mit dem anorganischen Kation langsam zugetropft, wodurch die anorganisch-organische Hybridverbindung langsam auf dem lipophilen Partikelkern abgeschieden wird und diesen umhüllt. Gegebenenfalls können Zwischenschichten (z.B. Tenside wie Tocopherolphosphat oder Monododecylposphat) verwendet werden, um die Haftung und Abscheidung von hydrophiler Hybridverbindung auf dem lipophilen Partikelkern zu erhöhen und zu verbessern.

Sämtliche vorstehende Ausführungen bezüglich des erfindungsgemäßen Nanocontainers treffen auch für das erfindungsgemäße Verfahren zur Herstellung des Nanocontainers zu.

Im Schritt (I) des erfindungsgemäßen Verfahrens wird eine Lösung der mindestens einen lipophilen Verbindung, wobei die lipophile Verbindung ein lipophiler, pharmazeutisch aktiver Wirkstoff und/oder ein lipophiles Nachweisreagenz ist, bereitgestellt. Als Lösungsmittel kann beispielsweise Methanol, Ethanol, 1-Propanol, 2-Propanol, Butanol, Tetrahydrofuran, Dioxan, Benzylalkohol, Dimethylsulfoxid, Acetonitril, Dimethylformamid, Aceton, Hexan, Dodekan, Toluol oder Gemische davon verwendet werden. Bevorzugte Lösungsmittel sind Ethanol, Benzylalkohol, Aceton, Tetrahydrofuran und Dimethylsulfoxid.

Gemäß einer weiteren Ausführungsform kann die Lösung der mindestens einen lipophilen Verbindung ferner einen lipophilen Hilfsstoff enthalten, beispielsweise Tocopherolphosphat oder Monodecylphosphat. Der lipophile Hilfsstoff kann die Stabilität des lipophilen Kerns erhöhen. Wird kein lipophiler Hilfsstoff zugesetzt, erhöht sich die Beladungsmenge an lipophiler Verbindung pro Nanocontainer erheblich.

Im Schritt (II) des erfindungsgemäßen Verfahrens wird die im Schritt (I) bereitgestellte Lösung dann in ein polares Lösungsmittel injiziert. Als polares Lösungsmittel können beispielsweise deionisiertes Wasser, wässrige NaCI-Lösungen, Ethanol, Dimethylsulfoxid oder Gemische davon verwendet werden. Bevorzugte Lösungsmittel sind deionisiertes Wasser oder wässrige NaCI-Lösungen.

Gemäß einer weiteren Ausführungsform der vorliegenden Erfindung muss der lipophile Hilfsstoff nicht in der im Schritt (I) bereitgestellten Lösung enthalten sein. Gegebenenfalls können ein oder mehrere lipophile Hilfsstoffe auch in dem polaren Lösungsmittel bereitgestellt werden.

Des Weiteren können ein oder mehrere chemische Verbindungen, beispielsweise Ionenverbindungen, in dem polaren Lösungsmittel enthalten sein. Beispielhaft kann hier Ammoniumacetat genannt werden, welches den pH-Wert des polaren Lösungsmittels stabilisiert.

Im Schritt (III) werden dann nacheinander, üblicherweise in dieser Reihenfolge, das organische Anion und das anorganische Kation, welche die anorganisch-organische Hybridverbindung zum Aufbau der Schale bilden, zugegeben.

Die Reaktionstemperatur des erfindungsgemäßen Verfahrens unterliegt keiner besonderen Beschränkung. Typischerweise wird ein Temperaturbereich zwischen -50°C und +90°C verwendet. Eine Kühlung mit Eis oder Trockeneis oder einer geeigneten Kühlflüssigkeit (z.B. gekühlt mit einem Kryostat) kann sinnvoll sein, um die Löslichkeit der Substanzen herabzusetzen. Vorzugsweise wird das Verfahren bei Raumtemperatur durchgeführt.

Nach Durchführung des Schrittes (III) fällt üblicherweise der gebildete erfindungsgemäße Nanocontainer aus bzw. liegt im verwendeten Lösungsmittel in Suspension vor. Nach dem Schritt (III) kann gegebenenfalls der Schritt (IV) erfolgen. Dieser optionale Schritt (IV) umfasst das Isolieren und/oder Aufreinigen des ausgefällten Nanocontainers. Dieses Isolieren und/oder Aufreinigen unterliegt keiner Einschränkung und kann durch alle geeigneten Verfahren erfolgen. Derartige Verfahren sind im Stand der Technik bekannt.

Vorzugsweise erfolgt das Isolieren und/oder Aufreinigen der Nanocontainer durch ein Verfahren, ausgewählt aus der Gruppe, bestehend aus Zentrifugationstechniken, Dialysetechniken, Phasentransfertechniken, Chromatographietechniken, Waschtechniken und Kombinationen davon. Die vorstehend genannten Verfahren können auch kombiniert und/oder mehrfach durchgeführt werden.

Ein weiterer Aspekt der vorliegenden Erfindung betrifft ein Nanopartikel, umfassend den erfindungsgemäßen Nanocontainer, funktionalisiert mit mindestens einem Element, ausgewählt aus der Gruppe, bestehend aus Antikörpern, Peptiden, 5-Aminolävulinsäure, Folsäurederivaten, Albuminderivaten, Sacchariden und Liganden, für die spezifische Bindung an Rezeptoren von Zellen. Dadurch kann ein gezielter Transport des Nanocontainers und eine gezielte Freisetzung der Wirkstoffe bzw. Nachweisreagenzien erreicht werden.

Ein weiterer Aspekt der vorliegenden Erfindung betrifft die Verwendung des erfindungsgemäßen Nanocontainers in der Behandlung von Infektionen durch Bakterien und/oder Viren, von entzündlichen Autoimmunreaktionen oder zur Behandlung von Tumoren.

Die Figuren zeigen:
Fig. 1 zeigt die erfindungsgemäße Struktur der Kern@Schale-Nanocontainer mit einem lipophilen Wirkstoff als Kern und einer anorganisch-organischen Hybridverbindung als Schale.
Fig. 2 zeigt die erfindungsgemäße Synthese von Kern@Schale-Nanocontainer mit der Bildung des lipophilen Kerns und der hydrophilen Schale am Beispiel von CBD@[ZrO]²⁺[FMN]²⁻, wobei CBD: Cannabidiol den Kern und FMN: Flavinmononuklid die Schale bilden.

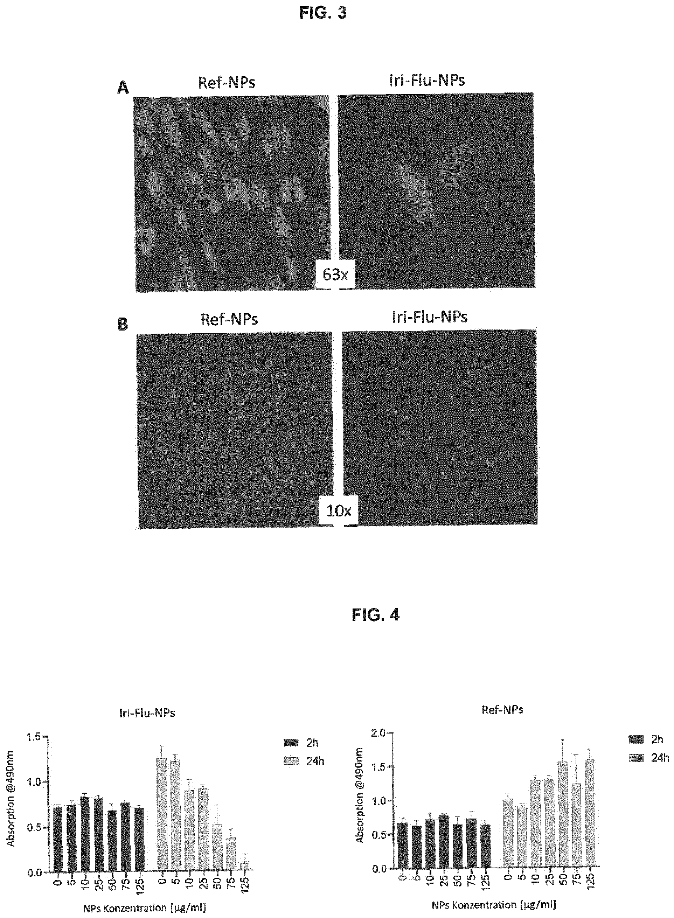

Fig. 3 zeigt Mikroskopie-Aufnahmen gemäß nachstehendem Ausführungsbeispiel 2, welche die Internalisierung der Kern@Schale-Nanocontainer durch Pankreastumorzellen zeigt. 25000 Zellen (13000 Zellen cm⁻²) wurden auf Cover-slips ausplattiert. Nach 24 h wurden die Zellen mit den ITC@[ZrO]²⁺[FLU]²⁻-Kern@Schale-Nanocontainer und den Referenz-Nanocontainern (50 µL/500 µL Medium) für 48 h behandelt, mit DAPI (Nuklei-Färbung) gegengefärbt, fixiert und unter einem konfokalen Fluoreszenzmikroskop analysiert. (A) zeigt eine Nahaufnahme unter 63-facher Vergrößerung; die Nanocontainer (der DUT-546 Farbstoff) sind in den hellen Bereichen dargestellt, die dunklen Bereiche zeigen die mit DAPI gefärbten Nuklei. (B) zeigt eine Übersichtsaufnahme (10-fache Vergrößerung), die mit DAPI gefärbte Nuklei zeigt; die toxische Wirkung der ITC@[ZrO]²⁺[FLU]²⁻-Kern@Schale-Nanocontainer (kaum Zellen vorhanden) im Vergleich zu den Referenz-Nanocontainern (ein konfluenter Zellteppich) ist deutlich.
Fig. 4 zeigt Diagramme gemäß nachstehendem Ausführungsbeispiel 3, worin 10000 Brustkrebszellen (pH8N8) mit einer Konzentration von 30000 Zellen cm⁻² ausplattiert und mit steigenden Konzentrationen der ITC@[ZrO]²⁺[FLU]²⁻-Kern@Schale-Nanocontainer und der Referenz-Nanocontainern, welche keinen pharmazeutisch aktiven Wirkstoff enthalten, behandelt wurden. Die Wirksamkeit auf die Hemmung des Zellwachstums wurde unter Verwendung des CellTiter 96^{®} AQueous One Solution Cell Proliferation Assays vor (2 h) und nach Behandlung (24 h) gemessen. Das Experiment wurde als Triplikat durchgeführt, die Balken stellen die Mittelwerte und die Fehlerbalken die Standardabweichung dar.

### Beispiele

Die folgenden Beispiele dienen der Veranschaulichung der vorliegenden Erfindung, ohne jedoch hierauf beschränkt zu sein.

### Ausführungsbeispiel 1.1: Herstellung von CBD@[ZrO]²⁺[FMN]²⁻- Nanocontainern:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Cannabidiol (CBD; 2,3 mg, 7,2 µmol) in 0,2 mL Ethanol wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit CBD-Nanopartikeln, die den späteren Nanocontainerkern darstellen, ist kolloidal für etwa 1 h stabil.

Die Suspension der CBD-Nanopartikeln wird im nächsten Schritt über 2 min unter intensiven Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Flavinmononukleotid-Lösung (FMN, Dinatriumsalz; 3,2 mg, 7,0 µmol) zugespritzt und für weitere 10 min gerührt. Auf diese Weise wird [ZrO]²⁺[FMN]²⁻ als anorganisch-organische Hybridverbindung als Schale auf dem CBD-Kern abgeschieden, so dass CBD@[ZrO]²⁺[FMN]²⁻-Kern@Schale-Nanocontainer gebildet werden. Die resultierende gelbe Suspension wird zentrifugiert (10 min, 25000 U min⁻¹), und die CBD@[ZrO]²⁺[FMN]²⁻-Kern@Schale-Nanocontainer werden in deionisiertem Wasser resuspendiert.

Die CBD@[ZrO]²⁺[FMN]²⁻-Kern@Schale-Nanocontainer mit Entzündungshemmer und Fluoreszenzfarbstoff sind als Suspension in Wasser kolloidal sehr stabil. Sie weisen gemäß Elektronenmikroskopie einen durchschnittlichen Durchmesser von 50 nm auf, wobei der Kern einen Durchmesser um 20 nm und die Schale eine Dicke von etwa 15 nm aufweisen.

### Ausführungsbeispiel 1.2: Herstellung von ERB@[ZrO]²⁺[FdUMP]²⁻-Nanocontainern:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Epirubicin (ERB; 3,9 mg, 7,2 µmol) in 0,2 mL Ethanol wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit ERB-Nanopartikeln, die den späteren Nanocontainerkern darstellen, ist kolloidal für etwa 1 h stabil.

Die Suspension der ERB-Nanopartikel wird im nächsten Schritt über 2 min unter intensivem Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13.000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer 5-Fluor-2'-deoxyuridin-5'-monophosphat-Lösung (FdUMP, Dinatriumsalz; 2,3 mg, 7,0 µmol) zugespritzt und für weitere 10 min gerührt. Auf diese Weise wird [ZrO]²⁺[FdUMP]²⁻ als anorganisch-organische Hybridverbindung als Schale auf dem ERB-Kern abgeschieden, so dass ERB@[ZrO]²⁺[FdUMP]²⁻-Kern@Schale-Nanocontainer gebildet werden. Die resultierende Suspension wird zentrifugiert (10 min, 25000 U min⁻¹), und die ERB@[ZrO]²⁺[FdUMP]²⁻-Kern@Schale-Nanocontainer werden in deionisiertem Wasser resuspendiert.

Die ERB@[ZrO]²⁺[FdUMP]²⁻-Kern@Schale-Nanocontainer mit zwei chemotherapeutischen Wirkstoffen sind als Suspension in Wasser kolloidal sehr stabil. Sie weisen gemäß Elektronenmikroskopie einen mittleren Durchmesser um 60 nm auf, wobei der Kern einen mittleren Durchmesser um 30 nm und die Schale eine Dicke von etwa 15 nm aufweisen.

Durch die Zugabe von Lumogen Rot zur ERB-Lösung (1 mol-% bezogen auf die Menge an ERB) und/oder Zugabe von Dynomics-647-uridintriphosphat zur FdUMP-Lösung (0,1 mol-% bezogen auf die Menge an FdUMP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.3: Herstellung von PAC@[Gd(OH)]²⁺[GemP]²⁻-Nanocontainer:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Paclitaxel (PAC; 6,1 mg, 7,2 µmol) in 0,2 mL Tetrahydrofuran wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit PAC-Nanopartikeln, die den späteren Nanocontainerkern darstellen, ist kolloidal für etwa 1 h stabil.

Die Suspension der PAC-Nanopartikel wird im nächsten Schritt über 2 min unter intensivem Rühren zu 9 mL einer GdCl₃-Lösung (Hexahydrat; 14,1 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Gemcitabinmonophosphat-Lösung (GemP, Dinatriumsalz; 2,4 mg, 7,0 µmol) zugespritzt und für weitere 10 min gerührt. Auf diese Weise wird [Gd(OH)]²⁺[GemP]²⁻ als anorganisch-organische Hybridverbindung als Schale auf dem PAC-Kern abgeschieden, so dass PAC@[Gd(OH)]²⁺[GemP]²⁻-Kern@Schale-Nanocontainer gebildet werden. Die resultierende Suspension wird zentrifugiert (10 min, 25000 U min⁻¹), und die PAC@[Gd(OH)]²⁺[GemP]²⁻-Kern@Schale-Nanocontainer werden in deionisiertem Wasser resuspendiert.

Die PAC@[Gd(OH)]²⁺[GeMP]²⁻-Kern@Schale-Nanocontainer mit zwei chemotherapeutischen Wirkstoffen sind als Suspension in Wasser kolloidal sehr stabil. Sie weisen gemäß Elektronenmikroskopie einen mittleren Durchmesser um 50 nm auf, wobei der Kern einen mittleren Durchmesser um 20 nm und die Schale eine Dicke von etwa 15 nm aufweisen.

Durch die Zugabe von Lumogen Grün zur PAC-Lösung (1 mol-% bezogen auf die Menge an PAC) und/oder Zugabe von Dynomics-647-uridintriphosphat zur GemP-Lösung (0,1 mol-% bezogen auf die Menge an GemP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.4: Herstellung von AMT@[ZrO]²⁺[RemP]²⁻-Nanocontainern:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Amantadin (AMT; 1,1 mg, 7,2 µmol) in 0,4 mL Tetrahydrofuran wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit AMT-Nanopartikeln, die den späteren Nanocontainerkern darstellen, ist kolloidal für etwa 1 h stabil.

Die Suspension der AMT-Nanopartikel wird im nächsten Schritt über 2 min unter intensivem Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Remdesivirphosphat-Lösung (RemP, Dinatriumsalz; 2,6 mg, 7,0 µmol) zugespritzt und für weitere 10 min gerührt. Auf diese Weise wird [ZrO]²⁺[RemP]²⁻ als anorganisch-organische Hybridverbindung als Schale auf dem AMT-Kern abgeschieden, so dass AMT@[ZrO]²⁺[RemP]²⁻-Kern@Schale-Nanocontainer gebildet werden. Die resultierende Suspension wird zentrifugiert (10 min, 25.000 U min⁻¹), und die AMT@[ZrO]²⁺[RemP]²⁻-Kern@Schale-Nanocontainer werden in deionisiertem Wasser resuspendiert.

Die AMT@[ZrO]²⁺[RemP]²⁻-Kern@Schale-Nanocontainer mit zwei antiviralen Wirkstoffen sind als Suspension in Wasser kolloidal sehr stabil. Sie weisen gemäß Elektronenmikroskopie einen mittleren Durchmesser um 40 nm auf, wobei der Kern einen mittleren Durchmesser um 20 nm und die Schale eine Dicke von etwa 10 nm aufweisen.

Durch die Zugabe von Lumogen Orange zur AMT-Lösung (1 mol-% bezogen auf die Menge an AMT) und/oder Zugabe von Dynomics-546-uridintriphosphat zur RemP-Lösung (0,1 mol-% bezogen auf die Menge an RemP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.5: Herstellung von ERB@[ZrO]²⁺[FdUMP]²⁻-Nanocontainern:

Tocopherolphosphat (Dinatriumsalz; 1,9 mg, 3,5 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Epirubicin (ERB; 7,8 mg, 14,4 µmol) in 0,4 mL Ethanol wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit ERB-Nanopartikeln, die den späteren Nanocontainerkern darstellen, ist kolloidal für etwa 1 h stabil.

Die Suspension der ERB-Nanopartikel wird im nächsten Schritt über 2 min unter intensivem Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer 5-Fluor-2'-deoxyuridin-5'-monophosphat-Lösung (FdUMP, Dinatriumsalz; 4,5 mg, 14,0 µmol) zugespritzt und für weitere 10 min gerührt. Auf diese Weise wird [ZrO]²⁺[FdUMP]²⁻ als anorganisch-organische Hybridverbindung als Schale auf dem ERB-Kern abgeschieden, so dass ERB@[ZrO]²⁺[FdUMP]²⁻-Kern@Schale-Nanocontainer gebildet werden. Die resultierende Suspension wird zentrifugiert (10 min, 25000 U min⁻¹), und die ERB@[ZrO]²⁺[FdUMP]²⁻-Kern@Schale-Nanocontainer werden in deionisiertem Wasser resuspendiert.

Die ERB@[ZrO]²⁺[FdUMP]²⁻-Kern@Schale-Nanocontainer mit zwei chemotherapeutischen Wirkstoffen sind als Suspension in Wasser kolloidal sehr stabil. Sie weisen gemäß Elektronenmikroskopie einen mittleren Durchmesser um 80 nm auf, wobei der Kern einen mittleren Durchmesser um 40 nm und die Schale eine Dicke von etwa 20 nm aufweisen.

Durch die Zugabe von Lumogen Rot zur ERB-Lösung (1 mol-% bezogen auf die Menge an ERB) und/oder Zugabe von Dynomics-647-uridintriphosphat zur FdUMP-Lösung (0,1 mol-% bezogen auf die Menge an FdUMP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.6: Herstellung von DCF@[GD(OH)]²⁺[BMP]²⁻-Nanocontainern:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Diclofenac (DCF, Natriumsalz; 2,3 mg, 7,2 µmol) in 0,2 mL Benzylalkohol wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit DCF-Nanopartikeln, die den späteren Nanocontainerkern darstellen, ist kolloidal für etwa 1 h stabil.

Die Suspension der DCF-Nanopartikel wird im nächsten Schritt über 2 min unter intensivem Rühren zu 9 mL einer GdCl₃-Lösung (Hexahydrat; 14,1 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Betamethasonphosphat-Lösung (BMP, Dinatriumsalz; 3,6 mg, 7,0 µmol) zugespritzt und für weitere 10 min gerührt. Auf diese Weise wird [Gd(OH)]²⁺[BMP]²⁻ als anorganisch-organische Hybridverbindung als Schale auf dem DCF-Kern abgeschieden, so dass DCF@[Gd(OH)]²⁺[BMP]²⁻-Kern@Schale-Nanocontainer gebildet werden. Die resultierende Suspension wird zentrifugiert (10 min, 25000 U min⁻¹), und die DCF@[Gd(OH)]²⁺[BMP]²⁻-Kern@Schale-Nanocontainer werden in deionisiertem Wasser resuspendiert.

Die DCF@[Gd(OH)]²⁺[BMP]²⁻-Kern@Schale-Nanocontainer mit zwei entzündungshemmenden Wirkstoffen sind als Suspension in Wasser kolloidal sehr stabil. Sie weisen gemäß Elektronenmikroskopie einen mittleren Durchmesser um 50 nm auf, wobei der Kern einen mittleren Durchmesser um 20 nm und die Schale eine Dicke von etwa 15 nm aufweisen.

Durch Zugabe von 3,3-Diethylthiadicarbocyaniniodid zur DCF-Lösung (1 mol-% bezogen auf die Menge an DCF) und/oder Zugabe von Flavinmononuklid zur BMP-Lösung (5,0 mol-% bezogen auf die Menge an BMP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.7: Herstellung von BDQ@[ZrO]²⁺[CLP]²⁻-Nanocontainern:

Tocopherolphosphat (Dinatriumsalz; 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Bedaquilin (BDQ; 4,0 mg, 7,2 µmol) in 0,3 mL Dimethylsulfoxid wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit BDQ-Nanopartikeln, die den späteren Nanocontainerkern darstellen, ist kolloidal für etwa 1 h stabil.

Die Suspension der BDQ-Nanopartikel wird im nächsten Schritt über 2 min unter intensivem Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Clindamycinphosphat-Lösung (CLP, Dinatriumsalz; 3,8 mg, 7,0 µmol) zugespritzt und für weitere 10 min gerührt. Auf diese Weise wird [ZrO]²⁺[CLP]²⁻ als anorganisch-organische Hybridverbindung als Schale auf dem BDQ-Kern abgeschieden, so dass BDQ@[ZrO]²⁺[CLP]²⁻-Kern@Schale-Nanocontainer gebildet werden. Die resultierende Suspension wird zentrifugiert (10 min, 25000 U min⁻¹), und die BDQ@[ZrO]²⁺[CLP]²⁻-Kern@Schale-Nanocontainer werden in deionisiertem Wasser resuspendiert.

Die BDQ@[ZrO]²⁺[CLP]²⁻-Kern@Schale-Nanocontainer mit zwei antibiotischen Wirkstoffen sind als Suspension in Wasser kolloidal sehr stabil. Sie weisen gemäß Elektronenmikroskopie einen mittleren Durchmesser um 40 nm auf, wobei der Kern einen mittleren Durchmesser um 20 nm und die Schale eine Dicke von etwa 10 nm aufweisen.

Durch Zugabe von 3,3'-Diethylthiadicarbocyaniniodid zur BDQ-Lösung (1 mol-% bezogen auf die Menge an BDQ) und/oder Zugabe von Dynomics-647-uridintriphosphat zur CLP-Lösung (0,1 mol-% bezogen auf die Menge an CLP) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.8: Herstellung von ITC@[ZrO]²⁺[FLU]²⁻-Nanocontainern:

Tocopherolphosphat (Dinatriumsalz; 3,8 mg, 7,0 µmol) und Ammoniumacetat (36,1 mg; 468 µmol) werden in je 6 mL deionisiertem Wasser gelöst. Beide Lösungen werden vereinigt und mit einem Eisbad gekühlt. Eine Lösung von Irinotecan (ITC; 4,2 mg, 7,2 µmol) in 0,2 mL Benzylalkohol wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit ITC-Nanopartikeln, die den späteren Nanocontainerkern darstellen, ist kolloidal für etwa 1 h stabil.

Die Suspension der ITC-Nanopartikel wird im nächsten Schritt über 2 min unter intensivem Rühren zu 9 mL einer ZrOCl₂-Lösung (Octahydrat; 6,8 mg, 37,9 µmol) zugetropft. Diese Suspension wird für weitere 10 min gerührt. Nachfolgend wird zentrifugiert (10 min, 13000 U min⁻¹). Die Nanopartikel werden dann in 6 mL der oben beschriebenen Ammoniumacetat-Lösung mithilfe eines Ultraschallstabes (1 min) oder durch intensives Rühren resuspendiert. Nach 30 s werden über einen Zeitraum von 10 s 5 mL einer Fludarabin-Lösung (FLU, Dinatriumsalz; 2,9 mg, 7,0 µmol) zugespritzt und für weitere 10 min gerührt. Auf diese Weise wird [ZrO]²⁺[FLU]²⁻ als anorganisch-organische Hybridverbindung als Schale auf dem ITC-Kern abgeschieden, so dass ITC@[ZrO]²⁺[FLU]²⁻-Kern@Schale-Nanocontainer gebildet werden. Die resultierende Suspension wird zentrifugiert (10 min, 25000 U min⁻¹), und die ITC@[ZrO]²⁺[FLU]²⁻-Kern@Schale-Nanocontainer werden in deionisiertem Wasser resuspendiert.

Die ITC@[ZrO]²⁺[FLU]²⁻-Kern@Schale-Nanocontainer mit zwei chemotherapeutischen Wirkstoffen sind als Suspension in Wasser kolloidal sehr stabil. Sie weisen gemäß Elektronenmikroskopie einen mittleren Durchmesser um 60 nm auf, wobei der Kern einen mittleren Durchmesser um 30 nm und die Schale eine Dicke von etwa 15 nm aufweisen.

Durch Zugabe von Lumogen Rot zur ITC-Lösung (1 mol-% bezogen auf die Menge an ITC) und/oder Zugabe von Dynomics-546-uridintriphosphat zur FLU-Lösung (0,1 mol-% bezogen auf die Menge an FLU) können Nanocontainerkern und/oder Nanocontainerschale fluoreszenzmarkiert werden. Dem Fachmann ist dabei Absorptions- und Emissionsverhalten der jeweiligen Fluoreszenzfarbstoffe bekannt.

### Ausführungsbeispiel 1.9: Herstellung von PAC@[GdO]⁺[SN-38]⁻-Nanocontainern:

Tocopherolphosphat (Dinatriumsalz, 3,8 mg, 7,0 µmol) und Ammoniumacetat (36,1 mg, 468 µmol) werden in je 6 mL deionisiertem Wasser gelöst. Beide Lösungen werden vereinigt und mit einem Eisbad gekühlt. Eine Lösung von Paclitaxel (PAC; 6,1 mg, 7,2 µmol) in 0,2 mL DMSO wird unter intensivem Rühren injiziert. Während und nach der Injektion erfolgt eine zusätzliche Durchmischung der Lösung/Suspension mithilfe eines 10 s langen Ultraschallpulses. Die resultierende Suspension mit PAC-Nanopartikeln, die den späteren Partikelkern darstellen, ist kolloidal für etwa 1 h stabil.

Die Suspension der PAC-Nanopartikel wird im nächsten Schritt über 2 min unter intensivem Rühren zu 9 mL einer GdCl₃-Lösung (Hexahydrat, 14,1 mg, 37,9 µmol) zugetropft. Diese Suspension wird für weitere 10 min gerührt. Nachfolgend wird zentrifugiert (10 min, 13.000 U/min). Die Nanopartikel werden dann in 6 mL deionisiertem Wasser mit Hilfe eines Ultraschallstabes (1 min) oder durch intensives Rühren resuspendiert. Nach 30 s werden über einen Zeitraum von 10 s 5 mL einer alkalischen SN-38-Lösung (SN-38 als aktiver Metabolit von Irinotecan, pH 9, 2,7 mg, 7,0 µmol) zugespritzt und für weitere 10 min gerührt. Auf diese Weise wird [GdO]⁺[SN-38]⁻ als anorganisch-organische Hybridverbindung als Schale auf dem PAC-Kern abgeschieden, so dass PAC@[GdO]⁺[SN-38]⁻-Kern@Schale-Nanopartikel gebildet werden. Die resultierende Suspension wird zentrifugiert (25.000 U/min, 10 min), und die PAC@[GdO]⁺[SN-38]⁻-Kern@Schale-Nanopartikel werden in deionisiertem Wasser resuspendiert.

Die PAC@[GdO]⁺[SN-38]⁻-Kern@Schale-Nanopartikel mit zwei chemotherapeutischen Wirkstoffen sind als Suspension in Wasser kolloidal sehr stabil. Sie weisen gemäß Elektronenmikroskopie einen mittleren Durchmesser um 50 nm auf, wobei der Kern einen mittleren Durchmesser um 20 nm und die Schale eine Dicke von etwa 15 nm haben.

Durch Zugabe von Lumogen Grün zur PAC-Lösung (1 mol-% bezogen auf die Menge an PAC) und/oder Zugabe von Dynomics-647-uridintriphosphat zur SN-38-Lösung (0,1 mol-% bezogen auf die Menge an SN-38) können Partikelkern und/oder Partikelschale fluoreszenzmarkiert werden. Dem Fachmann ist dabei Absorptions- und Emissionsverhalten der jeweiligen Fluoreszenzfarbstoffe bekannt.

### Ausführungsbeispiel 2: Aufnahme der Kern@Schale-Nanocontainer

Das Ausführungsbeispiel 2 betrifft die Evaluation der Aufnahme der Kern@Schale-Nanocontainer durch Pankreastumorzellen. Hierbei wurden ITC@[ZrO]²⁺[FLU]²⁻-Kern@Schale-Nanocontainer, sowie entsprechende Referenz-Nanocontainer, die keinen pharmazeutisch aktiven Wirkstoff enthalten, verglichen.

Figur 3A zeigt eine stark vergrößerte Aufnahme/Internalisierung der Partikel nach 48 h Inkubation der Kern@Schale-Nanocontainer mit den Tumorzellen (Panc02 Zelllinie). Erwartungsgemäß führten die ITC@[ZrO]²⁺[FLU]²⁻-Kern@Schale-Nanocontainer zu einer starken Toxizität, wodurch nur noch wenige Zellen in dem Präparat vorhanden waren. Dieses ist in der Figur 3B (Übersichtsaufnahme, 10-fache Vergrößerung) noch deutlicher zu sehen.

### Ausführungsbeispiel 3: Wirksamkeit der Kern@Schale-Nanocontainer

Das Ausführungsbeispiel 3 zeigt die Wirksamkeit der Kern@Schale-Nanocontainer auf murine Mammakarzinomzellen. Die Messung erfolgte mit einem CellTiter 96^{®} AQueous One Solution Cell Proliferation Assay (MTS), das eine kolorimetrische Methode zur Bestimmung der Lebensfähigkeit von Zellen darstellt.

Wie die Fig. 4 zeigt, führt die Behandlung der Tumorzellen mit ITC@[ZrO]²⁺[FLU]²⁻-Kern@Schale-Nanocontainer zu einer konzentrationsabhängigen Toxizität, während die Inkubation mit den Referenz-Nanocontainer, die keinen pharmazeutisch aktiven Wirkstoff enthalten, keinen negativen Einfluss auf das Zellwachstum zeigt.

## Patentansprüche

1. Nanocontainer, umfassend
einen lipophilen Kern,
wobei der lipophile Kern mindestens eine lipophile Verbindung umfasst, ausgewählt aus einem lipophilen, pharmazeutisch aktiven Wirkstoff oder einem lipophilen Nachweisreagenz; und
eine hydrophile Schale, welche den lipophilen Kern umschließt,
wobei die hydrophile Schale aus einer anorganisch-organischen Hybridverbindung als lonenverbindung aufgebaut ist, wobei die anorganisch-organische Hybridverbindung aus einem anorganischen Metallkation, ausgewählt aus Mn²⁺, Sc³⁺, Y³⁺,La³⁺, Fe²⁺, Fe³⁺, [ZrO]²⁺, [HfO]²⁺, Bi³⁺, Gd³⁺ oder einem Lanthanoid Ln²⁺ oder Ln³⁺ oder einer hydratisierten Form dieser Kationen, und einem organischen Wirkstoffanion oder einem hydrophilen Nachweisreagenzanion, das jeweils mindestens eine Phosphat-, Phosphonat-, Sulfat-, Sulfonat-, Carbonat- oder Carboxylatgruppe als funktionelle Gruppe enthält, aufgebaut ist.

2. Nanocontainer nach Anspruch 1, wobei der mindestens eine lipophile, pharmazeutisch aktive Wirkstoff bzw. das lipophile Nachweisreagenz ausgewählt ist aus
der Gruppe der Antibiotika, bestehend aus Delamanid, Bedaquilin, Benzothiazinonen wie Benzothiazinon 043, Clofazimin, Rifampicin, Levofloxacin, Cefaclor, Cefpodoxim, Imipenem, Meropenem, Ciprofloxacin, Levofloxacin, Norfloxacin, Chloramphenicol, Trimethoprim, Azithromycin, Metronidazol, Linezolid, Tyrothricin, Rifabutin, Rifaximin, Fusidinsäure, Doxycyclin, Hydroxytamoxifen und Pantoprazol; oder
der Gruppe der Virostatika, bestehend aus Amantadin, Rimantadin, Penciclovir, Emivirin, FGI-106, Maraviroc, Sofosbuvir, Baloxavirmarboxil, Etravirin, Nevirapin, Atazanavir, Indinavir, Lopinavir, Nelfinavir, Tipranavir, Boceprevir, Telaprevir, Dolutegravir, Raltegravir und Tecovirimat; oder
der Gruppe der Chemotherapeutika, bestehend aus Ifosfamid, Paclitaxel, Docetaxel, Abraxan, Taxoter, Mechlorethamin, Erlotinib, Gefitinib, Imatinib, Vemurafenib, Cisplatin, Daunorubicin, Epirubicin, Lomustin, Vismodegib, Actinomycin D, Vinorelbin, Camptothecin, Topotecan, Irinotecan, Etoposid, Teniposid und Mercaptopurin; oder
der Gruppe der Entzündungshemmer, bestehend aus Cannabidiol, Triamcinolon, Budesonid, Diclofenac, Cortisol, Calcitriol, Leflunomid und nichtsteroidalen Antiphlogistika; oder
der Gruppe der Fluoreszenzfarbstoffe, bestehend aus Lumogen Rot, Lumogen Orange, Lumogen Gelb oder Lumogen Grün, Magnesiumphthalocyanin, Zinkphthalocyanin, 1,1'-Diethyl-4,4'-carbocyanin iodid, 3,3'-Diethylthiadicarbocyaniniodid, Magnesiumtetraphenylporphyrin und Phthalocyanin.

3. Nanocontainer nach Anspruch 1 oder 2, wobei die Masse des lipophilen, pharmazeutisch aktiven Wirkstoffes und/oder des lipophilen Nachweisreagenz 50 bis 100 Gew.-%, bezogen auf die Gesamtmasse des lipophilen Kerns, beträgt.

4. Nanocontainer nach einem der Ansprüche 1 bis 3, wobei der lipophile Kern weiter einen lipophilen Hilfsstoff umfasst.

5. Nanocontainer nach einem der Ansprüche 1 bis 4, wobei der lipophile Kern einen Durchmesser von 10 bis 150 nm aufweist.

6. Nanocontainer nach einem der Ansprüche 1 bis 5, wobei das hydrophile pharmazeutisch aktive Wirkstoffanion bzw. das hydrophile Nachweisreagenzanion in
der anorganisch-organischen Hybridverbindung ausgewählt ist aus
der Gruppe der Antibiotika, bestehend aus Clindamycinphosphat, Erythromycinphosphat, Tedizolidphosphat, CpG-Oligodesoxynukleotide, Fosfomycin, Moxalactam, Ceftriaxon, Amoxicillin, Phenoxymethylpenicillin, Aztreonam, Moxifloxacin und Bacitracin; oder
der Gruppe der Virostatika, bestehend aus Idoxuridinphosphat, Aciclovirphosphat, Penciclovirphosphat, Ganciclovirphosphat, Remdesivirphosphat, Galidesivirphosphat, Vidarabinphosphat, Ribavirinphosphat, Abacavirphosphat, Stavudinphosphat, Adefovir, Fosamprenavir, Fostemsavir, Tenofovir, Brincidofovir, Cidofovir, Foscarnet, Ivermectin, Bevirimat und Zanamivir, oder
der Gruppe der Chemotherapeutika, bestehend aus 5-Fluor-2'-desoxyuridin-5'-monophosphat, Gemcitabinmonophosphat, Gemcitabintriphosphat, Fludarabin, Pemetrexed, Methotrexat, Estramustinphosphat, Streptozotocinphosphat, Mitoxantronphosphat, Azacitidinphosphat, Cyclophosphamid Mustard, SN-38, Melphalan, Chlorambucil und Bendamustin; oder
der Gruppe der Entzündungshemmer, bestehend aus Betamethasonphosphat, Dexamethasonphosphat, Prednisolonphosphat, Sulfasalazin, Acetylsalicylat, Methotrexat, Ibuprofen, Naproxen und Ketoprofen; oder
der Gruppe der Fluoreszenzfarbstoffe, bestehend aus Phenylumbelliferonphosphat, Flavinmononukleotid, Methylfluorescinphosphat, Resorufinphosphat, Dynomics-546-uridintriphosphat, Dynomics-647-uridintriphosphat, Amaranth Rot, Chicago Sky Blue, Direktblau 71, Kongorot, Nuclear Fast Red, Acid Red 97 und Evens Blue.

7. Nanocontainer nach einem der Ansprüche 1 bis 6, wobei die Masse des hydrophilen, pharmazeutisch aktiven Wirkstoffanions und/oder des Nachweisreagenzanions 50 bis 90 Gew.-%, bezogen auf die Gesamtmasse der organischen Anionen in der Schale, beträgt.

8. Nanocontainer nach einem der Ansprüche 1 bis 7, wobei der Nanocontainer einen Durchmesser von 20 bis 300 nm aufweist.

9. Nanocontainer nach einem der Ansprüche 1 bis 8, wobei die mindestens eine lipophile Verbindung ein lipophiler, pharmazeutisch aktiver Wirkstoff ist.

10. Nanocontainer nach einem der Ansprüche 1 bis 9, wobei die anorganisch-organische Hybridverbindung aus einem vorstehend definierten anorganischen Metallkation und einem organischen Wirkstoffanion aufgebaut ist, um die hydrophile Schale zu bilden.

11. Verfahren zur Herstellung eines Nanocontainers nach einem der Ansprüche 1 bis 10 mittels Solvens-Antisolvens-Methode, umfassend die Schritte:
(I) Bereitstellen einer Lösung der mindestens einen lipophilen Verbindung, wobei die mindestens eine lipophile Verbindung ein lipophiler, pharmazeutisch aktiver Wirkstoff und/oder ein lipophiles Nachweisreagenz ist;
(II) Injizieren der im Schritt (I) bereitgestellten Lösung in ein polares Lösungsmittel; und
(III) Zugeben des organischen Anions und des anorganischen Kations, welche die anorganisch-organische Hybridverbindung zum Aufbau der hydrophilen Schale bilden.

12. Nanopartikel, umfassend
den Nanocontainer nach einem der Ansprüche 1 bis 10, funktionalisiert mit mindestens einem Element, ausgewählt aus der Gruppe, bestehend aus Antikörpern, Peptiden, 5-Aminolävuinsäure, Folsäurederivaten, Albuminderivaten, Sacchariden und Liganden, für die spezifische Bindung an Rezeptoren von Zellen.

13. Verwendung des Nanocontainers nach einem der Ansprüche 1 bis 10 und/oder des Nanopartikels nach Anspruch 12 zur Behandlung von Infektionen durch Bakterien und/oder Viren, von entzündlichen Autoimmunreaktionen oder zur Behandlung von Tumoren.
